# EUROPEAN PATENT SPECIFICATION

(11) **EP 1 438 945 B1**
(45) Date of publication and mention of the grant of the patent: **22.11.2006**
(21) Application number: 04425009.0
(22) Date of filing: 13.01.2004
(51) Int. Cl.: A61F 13/15

(54) **A work station for cutting and folding shaped sanitary napkins**
Eine Arbeitstation zum Ausschneiden und Falten von geformter Hygienevorlagen
Une station de travail pour découper et plier des couches formées

(30) Priority: 20.01.2003 IT cr20030001
(43) Date of publication of application: 21.07.2004
(73) Proprietor: DELTA s.r.l., 26012 Castelleone CR (IT)
(72) Inventor: Ghidelli, Luigi, 26012 Castelleone CR (IT)
(74) Representative: Mascioli, Alessandro

(56) References cited:
- EP-A- 0 749 742
- EP-A- 1 004 285
- GB-A- 333 857
- GB-A- 1 440 905
- US-A- 3 685 818

## Description

The invention relates to a work station for cutting and folding shaped sanitary napkins, that is usable in a production line for the manufacture of diapers, particularly of the shaped type for adult incontinence.

The diapers are normally made on production lines that comprise a plurality of work stations suitable for carrying out the phases of the productive process in sequence. Said work stations comprise operating devices and units such as support shafts for reels of raw materials, unwinding units, storage accumulators, units applying the absorbent material, glueing or sealing units, cutting units, positioning and final packaging units. Conveyor belts are normally used to transfer the semifinished product between the stations or the single operating units.

During the early production phases, the semifinished product consists of a continuous band comprising one or more layers of material, for example support material or absorbent material (fluff) glued or sealed together. At a certain step of the process, the diapers are cut from the band by a cutting station, the waste being eliminated; the single diapers are then sent to the last processing phases, which processing ends with folding and packaging.

The diapers, in particular those of the shaped type for adult incontinence, are voluminous and bulky, so they need to be folded before packaging both to facilitate packaging and to reduce the overall dimensions of the finished packages.

It is prior art to make two longitudinal folds along the sides of the diapers, and one or possible several transversal folds, in order to give them a rectangular shape that is particularly advantageous for packaging.

For this purpose, production lines are realized that comprise, amongst other things, units or devices for longitudinal folding of the edges, acting on the continuous band of semifinished product; a cutting unit positioned downstream of said units or devices for longitudinal folding, which obtains the single diapers from the band; further devices for carrying out the transversal folding.

However, this working sequence is not usable in production lines for the manufacture of shaped diapers. The cutting unit, in fact, acting on the band of semifinished product with the edges already folded, must perform a straight cut that is perpendicular to the band, and cannot perform a cut according to a generally curved trajectory to obtain shaped sanitary napkins both along the edges and at the ends. Moreover, the cut is generally irregular and aesthetically unsatisfactory because the longitudinal folds of the sides are almost never completely identical and symmetrical. This system is therefore generally used only to produce diapers of the non-shaped type for children but not for shaped diapers for adults, for which a high quality is required also from the aesthetic point of view.

To produce shaped diapers, production lines are generally used wherein a cutting unit obtains the single diapers from the semifinished band, and a folding unit carries out only the transversal folding of the diapers obtained thereby.

In such a case, the cutting unit acts on the taut band and without any longitudinal folding of the edges, and can cut cleanly and precisely the desired curved shape; however, it is not possible to fold the edges of the diapers longitudinally. To perform this operation, it is in fact necessary for the edges to be taut and without wrinkles, which occurs on the continuous band, stretched between two conveying rollers, but not on the single diapers. The single diapers remain rather wide and with protruding, unfolded side bands; there is difficulty in packaging them exploiting all available space; more voluminous packages are obtained with rounded rather than square sides; storage room and transport costs are increased; the package may result bulky and inconvenient also for the purchaser.

A partial solution to this problem exists in the prior art, which essentially consists of longitudinally cutting the side profile of the diapers, continuously from the band of the semifinished product, then folding the sides of the band towards the inside, and finally dividing the continuous band into single diapers with straight cuts perpendicular to said band. However, it is clear that so obtained diapers are shaped only on the sides but not at the ends, with an unsatisfactory result from the point of view of aesthetics and product finish. For this reason, this system is little used.

The object of the present invention is to eliminate the drawbacks and disadvantages disclosed above.

The object of the invention is therefore to create a work station for cutting and folding shaped sanitary napkins that enables to realize any kind of shaped diapers, particularly adult incontinence diapers, and enables to fold them both longitudinally along the edges and transversally, so as to facilitate packaging, reduce the volume of the finished packages, optimise exploitation of storage room and reduce transport costs.

Another object of the invention is to obtain shaped diapers of a high quality and finish to meet the market demand.

A further object of the invention is to obtain high productivity without excessive processing waste.

These objects are achieved by the present invention as it is characterised by the claims, consisting of a work station for cutting and folding shaped sanitary napkins, usable in a production line for the manufacture of shaped diapers, particularly for adult incontinence, comprising:
a cutting unit suitable for obtaining single shaped diapers from a continuous band of semifinished product and characterised by :
   - a first conveyor belt located downstream of said cutting unit and operating in a conveying direction (A) :
   - a second conveyor belt, operating in the same direction at greater speed than said first conveyor belt and positioned immediately downstream thereof, in such a way that the diapers can pass directly from said first conveyor belt to said second conveyor belt;
   - suction means associated with said second conveyor belt, suitable for maintaining the diapers aspirated against the surface thereof;
   - folding means acting on the route of said second conveyor belt, which can make longitudinal folds parallel to said conveying direction towards the inside on both sides of the diapers, for obtaining folded lateral edges and an unfolded central portion on said diapers.

The main advantage of the invention consists in that it enables to obtain diapers of the required shape by means of said cutting unit that acts on the continuous band, and that it also enables to refold them along the edges thanks to longitudinal stretching which is generated by the acceleration at the passage from the first conveyor belt to the second conveyor belt and which is maintained thanks to said suction means.

Shaped diapers are obtained in this way both on the edges and on the ends with a high degree of finish, that can be folded into an almost perfectly rectangular compact shape; the volume of the finished packages is thus reduced with savings in storage and transport costs.

Further features and advantages of the invention will be shown in greater detail in the following disclosure with the help of drawings that show preferred embodiments that are illustrated by way of nonlimiting example.
Figure 1 is a schematic side view of a work station for the production of diapers of a shaped type for adult incontinence according to the invention.
Figures 2, 3, 4 illustrate respectively, in a schematic manner, cross-sections II-II, III-III and IV-IV of Figure 1.

With reference to the details illustrated in the Figures, the invention concerns a work station 1 for cutting and folding shaped diapers, particularly of the type for adult incontinence, essentially comprising a cutting unit 2, a first conveyor belt 3, a second conveyor belt 4, suction means 5 and folding means 6.

The cutting unit 2 is per se of a prior-art type, suitable for cutting single shaped diapers P from a continuous band of semifinished product, that is not illustrated, comprising for example a plurality of layers of support material and absorbent material glued or sealed together.

The first conveyor belt 3 is positioned to convey the diapers P from the cutting unit 2 to the second conveyor belt 4, in a direction A.

Said second belt 4 has a higher conveying speed than the first belt 3, and is next to it in such a way that the diapers P can pass directly from the first conveyor belt 3 to the second conveyor belt 4.

The suction means 5, associated with the conveyor belt 4, act by aspiring air from a plurality of holes that are not illustrated, being located on the surface of said conveyor belt 4. Said holes may be distributed along the entire width of the conveyor belt 4 or also advantageously only in the centre thereof.

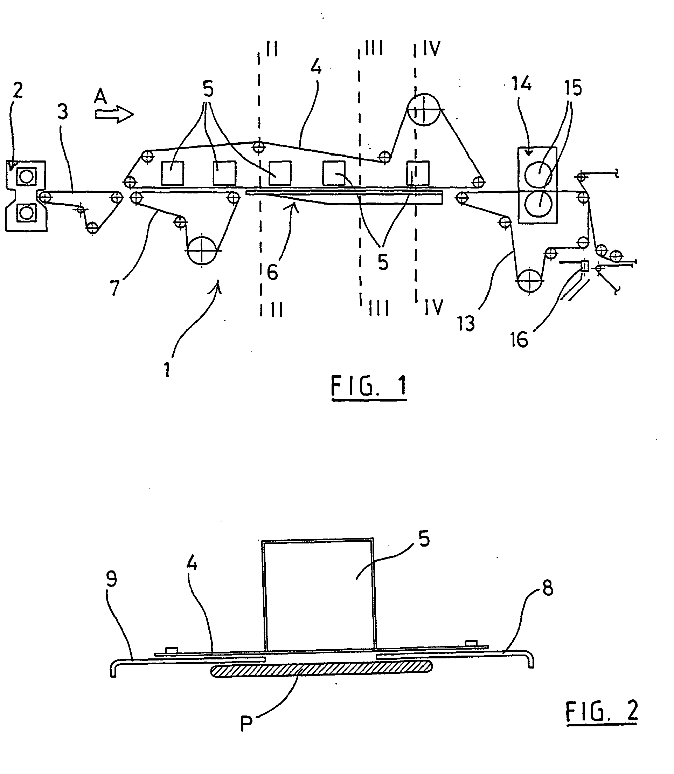

In the arrangement in Figure 1, which is the preferred one for the invention, the conveyor belt 4 is overturned, i.e. the diapers P face downwards and are held in place on said belt 4 only by the suction force that acts from the bottom to the top.

The station 1 also advantageously comprises another conveyor belt 7, located underneath the first intake section of the belt 4, in the manner shown schematically in Figure 1.

The folding means 6 acts on the diapers P whilst they are conducted by the conveyor belt 4, folding the side edges of the diapers towards the inside. In a preferred embodiment, said folding means 6 are made with two guide channels 8 and 9, obtained for example from folded sheet metal, positioned symmetrically in relation to the sides of the conveyor belt 4.

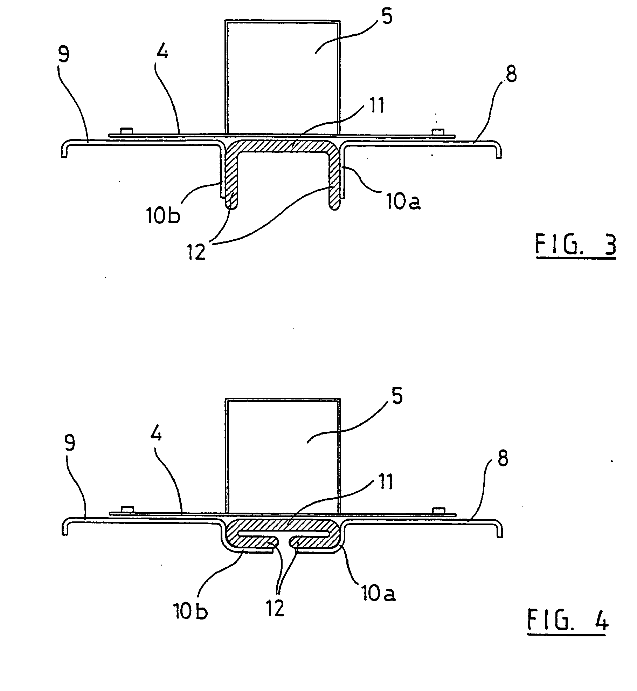

Said guide channels 8 and 9 respectively comprise a protruding part 10a and 10b, that is progressively folded, according to direction A, towards the inside of the belt 4, as illustrated in particular in the Figures 3 and 4.

An unfolded central portion 11 and folded side edges 12 are thus created on the diapers P, where said central portion 11 remains aspirated against the belt 4, and said side edges 12 remain free.

Advantageously, at the outfeed of the conveyor belt 4, a further conveyor belt 13 is provided that takes the diapers P to a pressing unit 14, essentially comprising two rollers 15. Lastly, a transversal folding unit or device is provided, for example comprising a rotating folder 16 synchronised with the conveying speed of the diapers P.

The invention operates as follows.

The diapers P, going from the conveyor belt 3 to the next, faster, conveyor belt 4, undergo longitudinal stretching that prevents the formation of folds or wrinkles, in particular along the side edges, and that is maintained by the suction means 5 that keeps the diapers aspirated against the conveyor belt 4.

The central portion 11 of the diapers P remains substantially flat, aspirated against the surface of the conveyor belt 4; the side edges 12, on the other hand, are folded longitudinally towards the inside by means of the protruding parts 10a and 10b of the channels 8 and 9, as schematically shown by the sequence of Figures 2, 3 and 4.
The longitudinal fold of the side edges 12 thereby obtained is stabilised by the pressing unit 14; lastly, the rotating folder 16 makes a transversal fold, for example half way along each diaper.

The disclosed invention enables, approximately, the overall dimensions of the packages to be reduced by about 20%, which saves on both storage and transport costs.

It is clear that the disclosed units and devices that are the subject of the invention are advantageously associated with a production line for the manufacture of diapers, and thus they are connected to a support structure and, where necessary, to appropriate mechanical transmission and/or electric power, compressed-air, vacuum supply means or other devices.

It is furthermore clear that the invention has been illustrated schematically without disclosing any further devices such as servomechanisms, sensors, controls, etc... that are part of the prior art in the sector of machines for diapers and sanitary napkins, and which will be provided according to requirements without the protection aims of the claims being thereby abandoned.

## Claims

1. A work station (1) for cutting and folding shaped sanitary napkins, that is usable in a production line for the manufacture of shaped diapers, particularly for adult incontinence, comprising a cutting unit (2) suitable for obtaining single shaped diapers (P) from a continuous band of semifinished product; and **characterised by** :
- a first conveyor belt (3) located downstream of said cutting unit (2), and operating in a conveying direction (A);
- a second conveyor belt (4), operating in said conveying direction (A) at greater speed than said first conveyor belt (3), and positioned immediately downstream thereof, in such a way that said diapers (P) can pass directly form said first conveyor belt (3) to said second conveyor belt (4);
- suction means (5) associated with said second conveyor belt (4), suitable for maintaining the diapers (P) aspirated against the surface thereof;
- folding means (6) acting on the route of said second conveyor belt (4), which can make longitudinal folds parallel to said conveying direction towards the inside on both sides of said diapers (P), for obtaining folded, lateral edges (12) and an unfolded, central portion (11) on said diapers (P).

2. A work station according to claim 1, **characterised in that** said suction means (5) can act only on said central portion (11) of the diapers (P), after folding made by said folding means (6).

3. A work station according to claim 1, **characterised in that** said second conveyor belt (4) is of the overturned type and said suction means (5) act by aspirating air from the bottom to the top, to keep the diapers (P) suspended on said second conveyor belt (4).

4. A work station according to claim 1, **characterised in that** said folding means (6) comprise two guide channels (8, 9), that extend on the sides of said second conveyor belt (4), suitable for making said longitudinal folds on the diapers (P).

5. A work station according to claim 4, **characterised in that** each of said guide channels (8, 9) comprise a protruding part (10a, 10b), progressively folded, according to the conveying direction (A) towards the inside of said conveyor belt (4).

6. A work station according to claim 1, **characterised in that** it also comprises a pressing unit (14), downstream said folding means (6).

7. A work station according to claim 1, **characterised in that** it also comprises at least one transversal folding device (16) of the diapers (P), placed downstream said folding means (6).

8. A production line for the manufacture of sanitary napkins, **characterised in that** it comprises a work station according to one or more of the previous claims.

## Patentansprüche

1. Arbeitsstation (1) für das Schneiden und Falten von geformten Binden, verwendbar in einer Produktionslinie für geformte Windeln insbesondere des Typs gegen Inkontinenz bei Erwachsenen, die ein Schneideaggregat (2) umfasst, das dazu dient einzelne geformte Windeln (P) von einem Endlosband des Halbfertigprodukts zu erhalten, die sich durch das Folgende auszeichnet:
- Ein erstes Transportband (3) unterhalb des genannten Schneideaggregats (2) und in einer Transportrichtung tätig (A);
- Ein zweites Transportband (4), mit einer höheren Geschwindigkeit als das genannte erste Transportband (3), in der genannten Transportrichtung tätig (A) und sofort unterhalb desselben positioniert, so dass die genannten Windeln (P) direkt von dem genannten ersten Transportband (3) auf das genannte zweite Transportband (4) übergehen können;
- Ansauggeräte (5) verbunden mit dem genannten zweiten Transportband (4), die dazu dienen, die Windeln (P) gegen die Oberfläche desselben angesaugt zu halten;
- Faltgeräte (6), die den Verlauf des genannten zweiten Transportbands (4) entlang tätig sind und Längsfalten erstellen können, die parallel zur genannten Transportrichtung liegen und nach innen hin auf beiden Seiten der genannten Windeln (P), um an den genannten Windeln (P) umgebogene Seitenränder (12) und einen nicht umgebogenen zentralen Teil zu erhalten.

2. Arbeitsstation entsprechend dem Patentanspruch 1, **dadurch** charakterisiert, dass die genannten Ansauggeräte (5) nur auf den genannten zentralen Teil (11) der Windeln (P) einwirken können, nachdem das Falten seitens der genannten Faltgeräte (6) erfolgte.

3. Arbeitsstation entsprechend dem Patentanspruch 1, **dadurch** charakterisiert, dass das genannte zweite Transportband (4) auf dem Kopf steht und die genannten Ansauggeräte (5) tätig sind, indem sie die Luft von unten nach oben ansaugen, um die Windeln (P) in der Schwebe an diesem genannten zweiten Transportband zu halten (4).

4. Arbeitsstation entsprechend dem Patentanspruch 1, **dadurch** charakterisiert, dass die genannten Faltgeräte (6) zwei Führungskanäle (8, 9) umfassen, die sich auf den Seiten des genannten zweiten Transportbands (4) erstrecken und dazu dienen, die genannten Längsfalten an den Windeln (P) zu erstellen.

5. Arbeitsstation entsprechend dem Patentanspruch 4, **dadurch** charakterisiert, dass jeder der genannten Führungskanäle (8, 9) einen herausragenden Teil (10a, 10b) umfasst, der progressiv gemäß der Transportrichtung (A) auf das Innere des genannten Transportbands (4) hin gefaltet ist.

6. Arbeitsstation entsprechend dem Patentanspruch 1, **dadurch** charakterisiert, dass sie auch ein Pressaggregat umfasst (14), das sich unterhalb der genannten Faltgeräte befindet (6).

7. Arbeitsstation entsprechend dem Patentanspruch 1, **dadurch** charakterisiert, dass sie auch mindestens eine Querfaltvorrichtung (16) der Windeln (P) umfasst, die sich unterhalb der genannten Faltgeräte befindet (6)

8. Produktionslinie von Binden, die sich **dadurch** auszeichnet, dass sie eine Arbeitsstation gemäß einer oder mehrerer der vorausgehenden Patentansprüche umfasst.

## Revendications

1. Station de travail (1) pour la découpe et le pliage de serviettes hygiéniques de type façonné, utilisable dans une ligne de production de couches façonnées particulièrement du type incontinence adultes, comprenant un groupe de découpe (2) apte à obtenir des couches individuelles façonnées (P) d'une bande continue de semi-fini, et **caractérisée par**:
- un premier tapis convoyeur (3) situé en aval de ce groupe de découpe (2), et opérant dans une direction de transport (A);
- un deuxième tapis convoyeur (4), opérant dans ladite direction de transport (A) à une vitesse supérieure dudit premier tapis convoyeur (3), et positionné immédiatement en aval de celui-ci, de sorte que ces couches (P) puissent passer directement dudit premier tapis convoyeur (3) audit deuxième tapis convoyeur (4);
- moyens d'aspiration (5) associés audit deuxième tapis convoyeur (4), aptes à maintenir les couches (P) aspirées contre la surface de celui-ci;
- moyens de pliage (6) agissant le long du parcours dudit deuxième tapis convoyeur (4), qui peuvent réaliser des pliages longitudinaux parallèles à cette direction de transport et vers l'intérieur sur les deux côtés desdites couches (P), pour obtenir sur lesdites couches (P) des bords latéraux repliés (12) et une portion centrale (11) non repliée.

2. Station de travail selon la revendication 1, **caractérisée par le fait que** lesdits moyens d'aspiration (5) peuvent agir seulement sur ladite portion centrale (11) des couches (P), après le pliage effectué par lesdits moyens de pliage (6).

3. Station de travail selon la revendication 1, **caractérisée par le fait que** ledit deuxième tapis convoyeur (4) est du type renversé et lesdits moyens d'aspiration (5) agissent en aspirant de l'air du bas vers le haut, pour maintenir les couches (P) suspendues audit deuxième tapis convoyeur (4).

4. Station de travail selon la revendication 1, **caractérisée par le fait que** lesdits moyens de pliage (6) comprennent deux canaux de guidage (8, 9), sur les côtés dudit deuxième tapis convoyeur (4), et sont aptes à réaliser lesdits pliages longitudinaux sur les couches (P).

5. Station de travail selon la revendication 4, **caractérisée par le fait que** chacun desdits canaux de guidage (8, 9) comprend une partie saillante (10a, 10b), pliée progressivement selon la direction de transport (A) vers l'intérieur dudit tapis convoyeur (4).

6. Station de travail selon la revendication 1, **caractérisée par le fait qu'**elle comprend également un groupe de pressage (14), situé en aval desdits moyens de pliage (6).

7. Station de travail selon la revendication 1, **caractérisée par le fait qu'**elle comprend aussi au moins un dispositif de pliage transversal (16) des couches (P), situé en aval desdits moyens de pliage (6)

8. Ligne de production de serviettes hygiéniques, **caractérisée par le fait qu'**elle comprend une station de travail selon une ou plusieurs des revendications précédentes.
